(19) Europäisches Patentamt
European Patent Office
Office européen des brevets

(11) Publication number: **0 200 305 B2**

(12) **NEW EUROPEAN PATENT SPECIFICATION**

(45) Date of publication of the new patent specification: **31.08.94**

(51) Int. Cl.5: **A61K 7/06**, A61K 7/08

(21) Application number: **86301362.9**

(22) Date of filing: **26.02.86**

(54) **Anti-dandruff shampoos.**

(30) Priority: **28.02.85 US 706576**

(43) Date of publication of application:
**05.11.86 Bulletin 86/45**

(45) Publication of the grant of the patent:
**27.03.91 Bulletin 91/13**

(45) Mention of the opposition decision:
**31.08.94 Bulletin 94/35**

(84) Designated Contracting States:
**AT BE CH DE FR GB IT LI LU NL**

(56) References cited:
**EP-A- 0 034 846**
**EP-A- 0 108 517**
**EP-A- 0 136 914**
**EP-A- 0 158 481**

**HAPPI HOUSEHOLD & PERSONAL PRODUCTS INDUSTRY, vol. 18, no. 2, February 1981, page 36, Ramsey, New Jersey, US; "Happi formulary"**

**CHEMICAL ABSTRACTS, vol. 96, no. 12, March 1982, page 393, abstract no. 91483f, Columbus, Ohio, US; & JP-A-81 133 400 (NIHON SURFACTANTS INDUSTRY CO., LTD) 19-10-1981**

(73) Proprietor: **THE PROCTER & GAMBLE COMPANY**
**One Procter & Gamble Plaza**
**Cincinnati Ohio 45202 (US)**

(72) Inventor: **Wetzel, Thomas Andrew**
**5367 Sanrio Court**
**Cincinnati Ohio 45239 (US)**

(74) Representative: **Brooks, Maxim Courtney et al**
**Procter & Gamble Limited**
**Whitley Road**
**Longbenton**
**Newcastle-upon-Tyne NE12 9TS (GB)**



Rank Xerox (UK) Business Services
(3.10/3.09/3.3.3)

CHEMICAL ABSTRACTS, vol. 99, no. 16, October 1983, page 342, abstract no. 128155r, Columbus, Ohio, US; & JP-A-58 29 900 (JOHNSON CO., LTD) 22-02-1983

Chemical Abstract 96:91.483f et brevet japonais JP 81 133,400 (Nihon surfactants)

Brevet japonais 150.294/80 (Lion) traduction en anglais

Chemical Abstract 99 : 128 155r et JOHNSON Co., JP 83 29,900

Derwent ref. 82-94137E LION CORPORATION, JP 82 156410

Article L 658.3 code de la Santé Publique

Analyse de l'acide stéarique (AK70) utilisé par Chimex

## Description

The present invention is related to antidandruff lotion shampoo compositions which possess good stability with respect to separation of the components and suspension of the antidandruff agent.

Lotion shampoos, both antidandruff as well as nondandruff types, are disclosed in the art. US-A-3,917,817, discloses a shampoo composition containing a piperazine based cationic polymer, 10% sodium alkyl sulfate, 4% lauryl monoethanolamide and 3% glycol distearate. US-A-4,013,787, discloses a similar composition. - Japanese Application, with Open for Public Inspection Number 60810, May 19, 1977 (Lion Fat & Oil), discloses shampoos containing 5% to 50% of an anionic surfactant, 1% to 10% of a fatty acid diethanol amide, 0.1% to 10% of an insoluble fine powder, and 1% to 10% of an ethyleneglycol ester. US-A-4,470,982 and the equivalent EP-A-0037876 disclose lotion antidandruff shampoos containing ethyleneglycol esters and an antidandruff agent.

While these references disclose compositions which contain components similar to those present in the compositions of the present invention, they do not suggest the advantages found by the present inventor for the compositions disclosed herein.

It is an object of the present invention, therefore, to provide shampoos containing specific ethyleneglycol - esters to serve as a suspending agent for a particulate antidandruff agent. The selected ethylene glycol esters can be used at lower levels than other ethylene glycol esters with the result being less interference with the deposition of the antidandruff active.

It is a further object of the present invention to provide effective antidandruff shampoos utilizing reduced levels of the antidandruff actives.

It is still a further object of the present invention to provide antidandruff shampoos exhibiting improved stability.

These and other objects will become readily apparent from the detailed description which follows.

All percentages and ratios herein are by weight unless otherwise indicated.

The present invention relates to a lotion shampoo composition comprising:

(A) from 5% to 30% by weight of a synthetic surfactant;

(B) from 0.5% to 4% by weight of an ethylene glycol diester wherein the diester is a mixture of stearate and palmitate groups containing from 55% to 80% stearate and from 20% to 45% palmitate;

(C) from 1% to 7% by weight of a $C_8$-$C_{14}$ fatty acid mono- or diethanolamide;

(D) from 0.1% to 3% by weight of a pyridinethione metal salt; and

(E) water.

wherein the viscosity of said compostion is from 1.5 to 4.5 Pa.•S (1500 to 4500 CPS), said viscosity being achieved by the inclusion, if necessary, of appropriate viscosity modifiers, said viscosity being measured using a Wells-Brooklield Viscosity being Cone model HBT-2000 using 0.5 $cm^3$ of product at 2 rpm and 27°C, and wherein said composition contains less than 10 ppm of anionic and nonionic clays and polymeric thickeners. The essential as well a optional components are described in the following paragraphs.

### Surfactant

An essential component of the present compositions is a synthetic surfactant. The surfactant, which may be selected from any of a wide variety of synthetic anionic, amphoteric, zwitterionic and nonionic surfactants, is present at a level of from 5% to 30%, preferably from 15% to 25%.

Synthetic anionic surfactants can be exemplified by the alkali metal salts of organic sulfuric reaction products having in their molecular structure an alkyl radical containing from 8-22 carbon atoms and a sulfonic acid or sulfuric acid ester radical (included in the term alkyl is the alkyl portion of higher acyl radicals). Preferred are the sodium, ammonium, potassium or triethanolamine alkyl sulfates, espacially those obtained by sulfating the higher alcohols ($C_8$-$C_{18}$ carbon atoms), sodium coconut oil fatty acid monoglyceride sulfates and sulfonates ; sodium or potassium salts of sulfuric acid esters of the reaction product of 1 mole of a higher fatty alcohol (e.g., tallow or coconut oil alcohols) and 1 to 12 moles of ethylene oxide; sodium or potassium salts of alkyl phenol ethylene oxide ether sulfate with 1 to 10 units of ethylene oxide per molecule and in which the alkyl radicals contain from 8 to 12 carbon atoms, sodium alkyl glyceryl ether sulfonates; ; the reaction product of fatty acids having from 10 to 22 carbon atoms esterified with isethionic acid and neutralized with sodium hydroxide; water soluble salts of condensation products of fatty acids with sarcosine; and other known in the art.

Zwitterionic surfactants can be exemplified by those which can be broadly described as derivatives of aliphatic quaternary ammonium, phosphonium, and sulfonium compounds, in which the aliphatic radicals

can be straight chain or branched, and wherein one of the aliphatic substituents contains from about 8 to 18 carbon atoms and one contains an anionic water-solubilizing group, e.g., carboxy, sulfonate, sulfate, phosphate, or phosphonate. A general formula for these compounds is:

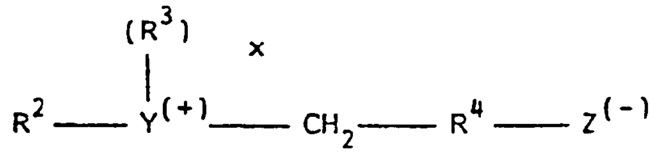

wherein $R^2$ contains an alkyl, alkenyl, or hydroxy alkyl radical of from 8 to 18 carbon atoms, from 0 to 10 ethylene oxide moieties and from 0 to 1 glyceryl moiety ; Y is selected from the group consisting of nitrogen, phosphorus, and sulfur atoms; $R^3$ is an alkyl or monohydroxyalkyl group containing 1 to 3 carbon atoms ; X is 1 when Y is a sulfur atom and 2 when Y is a nitrogen or phosphorus atom; $R^4$ is an alkylene or hydroxyalkylene of from 1 to 4 carbon atoms and Z is a radical selected from the group consisting of carboxylate, sulfonate, sulfate, phosphonate, and phosphate groups.

Examples include:
4-[N,N-di(2-hydroxyethyl)-N-octadecylammonio]-butane-1-carboxylate;
5-[S-3-hydroxypropyl-S-hexadecylsulfonio]-3-hydroxypentane-1-sulfate;
3-[P,P-diethyl-P-3,6,9-trioxatetradexocylphosphonio]-2-hydroxypropane-1-phosphate;
3-[N,N-dipropyl-N-3-dodecoxy-2-hydroxypropylammonio]-propane-1-phosphonate;
3-(N,N-dimethyl-N-hexadecylammonio)propane-1-sulfonate;
3-(N,N-dimethl-N-hexadecylammonio)-2-hydroxypropane-1-sulfonate;
4-[N,N-di(2-hydroxyethyl)-N-(2-hydroxydodecyl)ammonio]-butane-1-carboxylate;
3-[S-ethyl-S-(3-dodecoxy-2-hydroxypropyl)sulfonio]-propane-1-phosphate;
3-[P,P-dimethyl-P-dodecylphosphonio]-propane-1-phosphonate ; and
5-[N,N-di(3-hydroxypropyl)-N-hexadecylammonio]-2-hydroxypentane-1-sulfate.

Other zwitterionics such as betaines are also useful in the present invention. Examples of betaines useful herein include the high alkyl betaines such as coco dimethyl carboxymethyl betaine, lauryl dimethyl carboxymethyl betaine, lauryl dimethyl alphacarboxy-ethyl betaine, cetyl dimethyl carboxymethyl betaine, lauryl bis-(2-hydroxy-ethyl) carboxy methyl betaine, stearyl bis-(2-hydroxy-propyl) carboxymethyl betaine, oleyl dimethyl gamma-carboxypropyl betaine, lauryl bis-(2-hydroxypropyl) alpha-carboxyethl betaine, etc. The sulfobetaines may be represented by coco dimethyl sulfopropyl betaine, stearyl dimethyl sulfopropyl betaine, lauryl dimethyl sulfoethyl betaine, lauryl bis-(2-hydroxy-ethyl) sulfopropyl betaine and the like; amido betaines and amidosulfobetaines, wherein a $RCONH(CH_2)_3$ radical is attached to the nitrogen atom of the betaine are also useful in this invention. The amido betaines are preferred for use in some of the compositions of this invention.

Examples of amphoteric surfactants which can be used in the compositions of the present invention are those which can be broadly described as derivatives of aliphatic secondary and tertiary amines in which the aliphatic radical can be straight chain or branched and wherein one of the aliphatic substituents contains from 8 to 18 carbon atoms and one contains an anionic water solubilizing group, e.g., carboxy, sulfonate, sulfate, phosphate, or phosphonate. Examples of compounds falling within this definition are sodium 3-dodecylaminopropionate, sodium 3-dodecylaminopropane sulfonate, N-alkyitaurines such as the one prepared by reacting dodecylamine with sodium isethionate according to the teaching of US-A-2,658,072, N-higher alkyl aspartic acids such as those produced according to the teaching of US-A-2,438,091, and the products sold under the trade name "Miranol" and described in US-A-2,528,378.

Nonionic surfactants, which are preferably used in combination with an anionic, amphoteric or zwitterionic surfactant, can be broadly defined as compounds produced by the condensation of alkylene oxide groups (hydrophilic in nature) with an organic hydrophobic compound, which may be aliphatic or alkyl aromatic in nature. Examples of preferred classes of nonionic surfactants are:

1. The polyethylene oxide condensates of alkyl phenols, e.g., the condensation products of alkyl phenols having an alkyl group containing from 6 to 12 carbon atoms in either a straight chain or branched chain configuration, with ethylene oxide, the said ethylene oxide being present in amounts equal to 10 to 60 moles of ethylene oxide per mole of alkyl phenol. The alkyl substituent in such compounds may be derived from polymerized propylene, diisobutylene, octane, or nonane, for example.

2. Those derived from the condensation of ethylene oxide with the product resulting from the reaction of propylene oxide and ethylene diamine products which may be varied in composition depending upon the balance between the hydrophobic and hydrophilic elements which is desired. For example, compounds

containing from 40% to 80% polyoxyethylene by weight and having a molecular weight of from 5,000 to 11,000 resulting from the reaction of ethylene oxide groups with a hydrophobic base constituted of the reaction product of ethylene diamine and excess propylene oxide, said base having a molecular weight of the order of 2,500 to 3,000, are satisfactory.

3. The condensation product of aliphatic alcohols having from 8 to 18 carbon atoms, in either straight chain or branched chain configuration, with ethylene oxide, e.g., a coconut alcohol ethylene oxide condensate having from 10 to 30 moles of ethylene oxide per mole of coconut alcohol, the coconut alcohol fraction having from 10 to 14 carbon atoms.

4. Long chain tertiary amine oxides corresponding to the following general formula:

$$R_1R_2R_3N \rightarrow O$$

wherein $R_1$ contains an alkyl, alkenyl or monohydroxy alkyl radical of from 8 to 18 carbon atoms, from 0 to 10 ethylene oxide moieties, and from 0 to 1 glyceryl moiety, and $R_2$ and $R_3$ contain from 1 to 3 carbon atoms and from 0 to 1 hydroxy group, e.g., methyl, ethyl, propyl, hydroxy ethyl, or hydroxy propyl radicals. The arrow in the formula is a conventional representation of a semipolar bond. Examples of amine oxides suitable for use in this invention include dimethyl-dodecylamine oxide, oleyldi(2-hydroxyethyl) amine oxide, dimethyloctylamine oxide, dimethyl-decylamine oxide, dimethyl-tetradecylamine oxide, 3,6,9-trioxaheptadecyldiethylamine oxide, di(2-hydroxyethyl)-tetradecylamine oxide, 2-dode-coxyethyldimethylamine oxide, 3-dodecoxy-2-hydroxypropyldi(3-hydroxypropyl)amine oxide, dimethylhexadecylamine oxide.

5. Long chain tertiary phosphine oxides corresponding to the following general formula:

$$RR'R''P \rightarrow O$$

wherein R contains an alkyl, alkenyl or monohydroxyalkyl radical ranging from 8 to 18 carbon atoms in chain length, from 0 to 10 ethylene oxide moieties and from 0 to 1 glyceryl moiety and R' and R'' are each alkyl ormonohydroxyalkyl groups containing from 1 to 3 carbon atoms. The arrow in the formula is a conventional representation of a semipolar bond. Examples of suitable phosphine oxides are: dodecyl-dimethylphosphine oxide, tetradecyldimethylphosphine oxide, tetradecylmethylethylphosphine oxide, 3,6,9,-trioxaoctadecyldimethylphosphine oxide, cetyldimethylphosphine oxide, 3-dodecoxy-2-hydroxypropyldi(2-hydroxyethyl)phosphine oxide, stearyldimethylphosphine oxide, cetylethylpropyl-phosphine oxide, oleyldiethylphosphine oxide, dodecyldiethylphosphine oxide, tetradecyldiethyl-phosphine oxide, dodecyldipropylphosphine oxide, dodecyldi(hydroxymethyl)phosphine oxide, dodecyldi-(2-hydroxyethyl) phosphine oxide, tetradecylmethyl-2-hydroxypropylphosphine oxide, oleyldimethyl-phosphine oxide, 2-hydroxydodecyldimethylphosphine oxide.

6. Long chain dialkyl sulfoxides containing one short chain alkyl or hydroxy alkyl radical of 1 to 3 carbon atoms (usually methyl) and one long hydrophobic chain which contain alkyl, alkenyl, hydroxy alkyl, or keto alkyl radicals containing from 8 to 20 carbon atoms, from 0 to 10 ethylene oxide moieties and from 0 to 1 glyceryl moiety. Examples include:
octadecyl methyl sulfoxide, 2-ketotridecyl methyl sulfoxide, 3,6,9,-trioxaoctadecyl 2-hydroxyethyl sulfoxide, dodecyl methyl sulfoxide, oleyl 3-hydroxypropyl sulfoxide, tetradecyl methyl sulfoxide, 3-methoxytridecyl methyl sulfoxide, 3-hydroxytridecyl methyl sulfoxide, 3-hydroxy-4-dodecoxybutyl methyl sulfoxide.

Many additional nonsoap surfactants are described in McCUTCHEON'S, DETERGENTS AND EMULSIFIERS, 1983 Annual. published by Allured Publishing Corporation.

The above-mentioned surfactants can be used alone or in combination in the shampoo compositions of the present invention. The anionic surfactants, particularly the alkyl sulfates, the ethoxylated alkyl sulfates and mixtures thereof are preferred for use herein as well as the amido betaines.

## Ethylene Glycol Ester

The ethylene glycol esters found useful in the compositions of the present invention are diesters wherein the esters are a mixture of palmltate and stearate. The amount of stearate should be in the range of 55 to 80% with palmitate accounting for the remainder. The amount of stearate is preferably from 60% to 75%.

The amount of the ester useful in the present invention is from 0.5% to 4%, preferably from 1% to 4%. It has been surprisingly found that these levels provide for improved stability whereas materials not meeting

the ester requirement would have to be used at much higher levels to achieve similar stability.

Amide

The amide used in the present compositions are mono- and diethanolamides of fatty acids having from 8 to 14 carbon atoms. Preferred are coconut monoethanolamide, coconut diethanolamide and mixtures thereof. Other amides are those having multiple ethoxy groups such as PEG-3 lauramide.

The amide is present at a level of from 1% to 7%, preferably from 2% to 5%.

Antidandruff Agent

Another essential component of the present invention is a particulate pyridinethione antidandruff agent. Included among such agents are zinc pyridinethione and other 1-hydroxy pyridinethiones such as those disclosed in US-A-2,809,971, US-A-3,236,733, US-A-3,753,196, US-A-3,761,418, and US-A-4,379,753.

Preferred pyridinethione salts are those having a median particle diameter of at least 1.2 µm.

Also preferred are the metal salt pyridinethione salts of the type disclosed in US-A-4,379,753. Zinc pyridinethione is the preferred agent, particularly where its salt crystals are predominantly flat platelets which have a mean sphericity less than about 0.65, preferably between about 0.20 and about 0.65, and a individual median particle diameter of at least about 2 µm, expressed as the diameter of a sphere of equivalent volume.

The diameter of a sphere of equivalent volume, $d_v$, for a particle can be determined by a variety of sedimentation techniques which are based on Stokes' Law for the settling velocity of a particle in a fluid. Such techniques are described in Stockham, J.D. and Fochtman, E.G., Particle Size Analysis, Ann Arbor Science, 1978.

The sphericity of a particle is also described by Stockham and Fochtman at page 113 as

$$\psi = \left(\frac{d_v}{d_s}\right)^2$$

where $d_v$ is the diameter of a sphere of equivalent volume, supra, and $d_s$ is the diameter of a sphere of equivalent area. A technique for determining $d_s$ is the BET technique described by Stockham and Fochtman at page 122.

Since the sphericity of interest herein is the mean sphericity, the mean diameters are employed.

The particulate anti dandruff agent is present at a level of 0.1% to 3%, preferably from 0.3% to 2%.

Water

Water is the remaining essential component of the present compositions and is present at a level of from 50% to 80%, preferably from 60% to 75%. The viscosity of the present compositions is in the range of 1.5 to 4.5 Pa•s (1500 to 4500 CPS), preferably from 2.0 to 4.0 Pa•s (2000 to 4000 CPS). (Wells-Brookfield Viscometer/CP-40 Cone model HBT-2000/0.5 cc of product at 2 RPM, temperature 80 °F [27 °C]-). The viscosity is achieved by the inclusion if necessary of viscosity modifiers and hydrotropes as listed below.

Optional Components

The shampoos herein can contain a variety of nonessential optional components suitable for rendering such compositions more acceptable. Such conventional optional ingredients are well known to those skilled in the art, e.g., preservatives such as benzyl alcohol, methyl paraben, propyl paraben, methylisothiazolinone and imidazolidinyl urea ; thickeners and viscosity modifiers such as amine oxides, blok polymers of ethylene oxide and propylene oxide such as Pluronic (RTM) F88 offered by BASF Wyandotte, fatty alcohols such as cetearyl alcohol, sodium chloride, ammonium chloride, sodium sulfate, polyvinyl alcohol, propylene glycol and ethyl alcohol; hydrotropes such as xylene sulfonate; pH adjusting agents such as citric acid, succinic acid, phosphoric acid, sodium hydroxide, sodium carbonate, etc. ; perfumes ; dyes ; quaternary

ammonium compounds such as Polyquatemium 41; and, sequestering agents such as disodium ethylenediamine tetraacetate. Such agents generally are used individually at a level of from 0.01% to 10%, preferably from 0.01% to 5.0% by weight of the composition.

Another optional component found useful in the present compositions is a 1-hydroxy-2 pyridone of the type disclosed in US-A-3,883,545. Such agents when used in combination whith the aforedescribed pyridinethiones are particularly preferred. These auxiliary antidandruff agents are used at a level of from 0.1% to 2%, preferably from 0.2% to 1%.

The compositions herein are free of anionic and nonionic clays and polymeric thickeners. By "free" is meant less than 10 ppm.

## METHOD OF MANUFACTURE

Methods for manufacturing the present compositions are disclosed in Examples 1 and 11.

## INDUSTRIAL APPLICABILITY

The present compositions are used in a conventional manner for cleaning hair. From about 0.1 g to about 20 g of a composition is applied to hair that has been wetted, generally with water, worked through the hair and then rinsed out.

The following Examples further describe and demonstrate the preferred embodiments within the scope of the present invention. The Examples are given solely for the purpose of illustration and are not to be construed as limitations of the present invention as many variations thereof are possible without departing from its spirit and scope.

## EXAMPLE I

The following composition is representative of the present invention.

| Component | Weight % |
|---|---|
| Zinc pyridinethione (ZPT)[1] | 1.000 |
| Triethanolamine Alkyl Sulfate | 19.390 |
| Coconut Monoethanolamide | 3.000 |
| Ethylene Glycol Distearate[2] | 3.000 |
| Sodium Chloride | up to 2.500 to obtain desired viscosity |
| Propylene Glycol | 0.500 |
| Preservative | 0.033 |
| Perfume | 0.600 |
| Citric Acid | 0.65 |
| Dye (1% Aqueous Solution) | 0.30 |
| Water | qs 100% |

[1] ZPT having a median particle size of about 2.0 microns

[2] Ethylene glycol distearate wherein the ester groups are not pure stearate but a mixture of palmitate and stearate in the ratio of 35:65.

This composition is prepared by heating part of the water to 66°C to 88°C. The sodium chloride, citric acid and surfactant are added to the water while it is heating. After the desired temperature is obtained, the amide, dye and ethylene glycol distearate are added and mixing continues for 10 minutes. The solution is then cooled to 21°C to 49°C. After this cooler temperature is obtained, the zinc pyridinethione, the preservative, perfume and the remainder of the water is added and mixing continues for 5-10 more minutes.

EXAMPLE II

The following is another composition representative of the present invention.

| Component | Weight % |
|---|---|
| Ammonium Lauryl Sulfate | 9.270 |
| Ammonium Lauryl $(Ethoxy)_3$ Sulfate | 10.230 |
| Citric Acid | 0.160 |
| Ethylene Glycol Distearate[1] | 3.000 |
| Coconut Monoethanolamlde | 4.000 |
| Ammonium Xylene Sulfonate | 2.170 |
| Dye (1% Aqueous Solution) | 0.100 |
| Propylene Glycol | 0.750 |
| Zinc Pyridinethione (ZPT)[2] | 1.000 |
| Preservative | 0.027 |
| Perfume | 0.650 |
| Water | qs 100.000% |

[1] Same as Example I
[2] ZPT in platelet form having a median individual particle diameter of about 15 microns

The above composition is prepared by mixing part of the water, part of the surfactant, citric acid, amide, distearate and dye together in a mix tank and heating the mixture from 66°C to 88°C. The mixture is then cooled to from 21°C to 49°C. After this cooling has taken place, the perfume, zinc pyridinethione, preservative and the remainder of the water and surfactant are added. The total mixture is agitated until a homogeneous mixture was obtained.

EXAMPLE III

The following compositions were prepared and tested for their ability to suspend zinc pyridinethione.

| Component | Weight % | | | |
|---|---|---|---|---|
| | A | B | C | D |
| TEA Alkyl Sulfate | 19.400% | 19.400% | 19.400% | 19.400% |
| Sodium Chloride | 1.200 | 1.200 | 1.200 | 1.200 |
| Citric Acid | 0.650 | 0.650 | 0.650 | 0.650 |
| Coconut Monoethanolamide | 3.000 | 3.000 | 3.000 | 3.000 |
| Ethylene Glycol Distearate[1] | 3.000 | 3.000 | 3.000 | 3.000 |
| Perfume | 0.600 | 0.600 | 0.600 | 0.600 |
| Dye | 0.320 | 0.320 | 0.320 | 0.320 |
| Preservative | 0.033 | 0.033 | 0.033 | 0.033 |
| Zinc pyridinethione[2] (ZPT) | 1.000 | 1.000 | 1.000 | 1.000 |
| Water | qs 100% → | | | |

[1]-A EGDS having a stearate:palmitate ratio of 53:47
[1]-B EGDS having a stearate:palmitate ratio of 59:41
[1]-C EGDS having a stearate:palmitate ratio of 48:52
[1]-D EGDS having a stearate:palmitate ratio of 65:35
[2] ZPT having a median individual particle diameter of about 2 microns

These compositions were prepared in the manner described in Exemple I. After preparation the compositions were packed into bottles, compositions A and B into 113.49 (4 oz) bottles adn C and D into 198.59 (7 oz) bottles. Compositions A and B were stored at 43.3°C (110°F) for 3.5 months while compositions C and D were stored 48.9°C (120°F) for 1.5 months. After these storage periods the samples were frozen and the top one third of samples A and B and the top quarter of samples C and D was removed for analysis. The sections were thawed, mixed thoroughly and analyzed. In the comparison of A with B,B, within the scope of the present invention, had 1.01% ZPT while A, outside the scope of the present invention, had only 0.93% ZPT. In the comparison of C with D,D, within the scope of the present invention, had 1.00% ZPT while C, outside the scope, had only 0.95%ZPT. It is seen that the present compositions provide for marked improvement in the suspension of the ZPT.

**Claims**

**1.** A lotion shampoo composition comprising:
(A) from 5% to 30% by weight of a synthetic surfactant;
(B) from 0.5% to 4% by weight of an ethylene glycol diester wherein the diester is a mixture of stearate and palmitate groups containing from 55% to 80% stearate and from 20% to 45% palmitate;

(C) from 1% to 7% by weight of a $C_8$-$C_{14}$ fatty acid mono- or diethanolamide;
(D) from 0.1% to 3% by weight of a pyridinethione metal salt; and
(E) water,

wherein the viscosity of said composition is from 1.5 to 4.5 Pa•s (1500 to 4500 CPS), said viscosity being achieved by the inclusion, if necessary, of appropriate viscosity modifiers, said viscosity being measured using a Wells-Brookfiel Viscometer/CP-40 Cone model HBT-2000 using 0.5 $cm^3$ of product at 2 rpm and 27°C, and wherein said composition contains less than 10 ppm of anionic and nonionic clays and polymeric thickeners.

**2.** A shampoo composition according to Claim 1 wherein the surfactant is anionic.

**3.** A shampoo composition according to Claim 1 or 2 wherein the pyridinethione metal salt is zinc pyridinethione.

**4.** A shampoo composition according to any of Claims 1 to 3 wherein the level of surfactant is from 15% to 25% by weight.

**5.** A shampoo composition according to any of Claims 1 to 4 wherein the level of ethylene glycol diester is from 1% to 4% by weight.

**6.** A shampoo composition according to any of Claims 1 to 5 wherein the level of pyridinethione metal salt is from 0.3% to 2% by weight.

**7.** A shampoo composition according to any of Claims 1 to 6 wherein the surfactant is selected from alkyl sulfates, ethoxylated alkyl sulfates and mixtures thereof.

**8.** A shampoo composition according to any of Claims 1 to 7 which in addition contains a 1-hydroxy-2-pyridone compound.

**9.** A shampoo composition according to any of Claims 1 to 8 which in addition contains xylene sulfonate.

**Patentansprüche**

**1.** Lotionsshampoozusammensetzung, umfassend:

(A) von 5 Gew.-% bis 30 Gew.-% an einem synthetischen grenzflächenaktiven Mittel;
(B) von 0,5 Gew.-% bis 4 Gw.-% an einem Ethylenglykoldiester, worin der Diester ein Gemisch von Stearat- und Palmitatgruppen ist, welches von 55% bis 80 % Stearat und von 20 % bis 45 % Palmitat enthält;
(C) von 1 Gew.-% bis 7 Gew.-% an einem $C_8$-$C_{14}$-Fettsäuremono- oder -diethanolamid
(D) von 0,1 Gew.-% bis 3 Gew.-% an einem Pyridinthionmetallsalz; und
(E) Wasser,

worin die Viskosität der genannten Zusammensetzung von 1,5 bis 4,5 Pa.s (1500 bis 4500 cPs) beträgt, welche Viskosität, wenn notwendig, durch den Einschluß von geeigneten viskositätsmodifizierenden Mitteln erzielt wird, welche Viskosität unter Verwendung eines Wells-Brookfield Viscometers/CP-40 Konusmodell HBT-2000 mit 0,5 $cm^3$ Produkt bei 2 UpM und 27°C gemessen wird, und wobei die Zusammensetzung weniger als 10 ppm an anionischen oder nichtionischen Tonen und polymeren Verdickungsmitteln enthält.

**2.** Shampoozusammensetzung nach Anspruch 1, worin das grenzflächenaktive Mittel anionisch ist.

**3.** Shampoozusammensetzung nach Anspruch 1 oder 2, worin das Pyridinthionmetallsalz Zinkpyridinthion ist.

**4.** Shampoozusammensetzung nach einem der Ansprüche 1 bis 3, worin die Menge an grenzflächenaktivem Mittel von 15 Gew.-% bis 25 Gew.-% beträgt.

**5.** Shampoozusammensetzung nach einem der Ansprüche 1 bis 4, worin die Menge an Ethylenglykoldiester von 1 Gew.-% bis 4 Gew.-% beträgt.

6. Shampoozusammensetzung nach einem der Ansprüche 1 bis 5, worin die Menge an Pyridinthionmetallsalz von 0,3 Gew.-% bis 2 Gew.-% beträgt.

7. Shampoozusammensetzung nach einem der Ansprüche 1 bis 6, worin das grenzflächenaktive Mittel unter Alkylsulfaten, ethoxylierten Alkylsulfaten und Gemischen hiervon ausgewählt ist.

8. Shampoozusammensetzung nach einem der Ansprüche 1 bis 7, welche zusätzlich eine 1-Hydroxy-2-pyridon-Verbindung enthält.

9. Shampoozusammensetzung nach einem der Ansprüche 1 bis 8, welche zusätzlich Xylolsulfonat enthält.

**Revendications**

1. Composition de shampooing en lotion, comprenant :
   (A) de 5% à 30% en poids d'un surfactif synthétique ;
   (B) de 0,5% à 4% en poids d'un diester de l'éthylène glycol, le diester étant un mélange de groupes stéarate et palmitate, contenant de 55% à 80% de stéarate et de 20% à 45% de palmitate ;
   (C) de 1% à 7% en poids d'un mono- ou di- éthanolamide d'acide gras en $C_8$ à $C_{14}$ ;
   (D) de 0,1% à 3% en poids d'un sel de métal de pyridinethione ; et
   (E) de l'eau,

   la viscosité de ladite composition étant de 1,5 à 4,5 Pa.s (1500 à 4500 cps), ladite viscosité étant obtenue par l'inclusion, si nécessaire, de modificateurs appropriés de la viscosité, ladite viscosité étant mesurée à l'aide d'un viscosimètre Wells-Brookfield/CP-40 Cone, modèle HBT-2000, en utilisant 0,5 $cm^3$ de produit à 2 tours par minutes et 27 °C et dans laquelle ladite composition contient moins de 10 ppm d'argiles anioniques et non ioniques et d'épaississants polymères.

2. Composition de shampooing selon la revendication 1 dans laquelle le surfactif est anionique.

3. Composition de shampooing selon la revendication 1 ou 2, dans laquelle le sel de métal de pyridinethione est de la pyridinethione de zinc.

4. Composition de shampooing selon l'une quelconque des revendications 1 à 3, dans laquelle la proportion de surfactif est de 15% à 25% en poids.

5. Composition de shampooing selon l'une quelconque des revendications 1 à 4, dans laquelle la proportion du diester d'éthylène glycol est de 1% à 4% en poids.

6. Composition de shampooing selon l'une quelconque des revendications 1 à 5, dans laquelle la proportion du sel de métal de pyridinethione est de 0,3% à 2% en poids.

7. Composition de shampooing selon l'une quelconque des revendications 1 à 6, dans laquelle le surfactif est choisi parmi des alkyl sulfates, des alkyl sulfates éthoxylés et leurs mélanges.

8. Composition de shampooing selon l'une quelconque des revendications 1 à 7, qui contient en outre une 1-hydroxy-2-pyridone.

9. Composition de shampooing selon l'une quelconque des revendications 1 à 8, qui contient en outre du xylène sulfonate.